# EUROPEAN PATENT SPECIFICATION

(11) **EP 3 458 985 B1**
(45) Date of publication and mention of the grant of the patent: **03.12.2025**
(21) Application number: 16725878.9
(22) Date of filing: 17.05.2016
(51) Int. Cl.: G16H 10/60, G16H 40/67

(54) **METHOD, DEVICE AND SYSTEM FOR VERIFYING USER HEALTH DATA**
VERFAHREN, VORRICHTUNG UND SYSTEM ZUR VERIFIZIERUNG VON GESUNDHEITSDATEN
PROCÉDÉ, DISPOSITIF ET SYSTÈME DE VÉRIFICATION DE DONNÉES DE SANTÉ D'UN UTILISATEUR

(43) Date of publication of application: 27.03.2019
(73) Proprietor: Nokia Technologies Oy, 02610 Espoo (FI)
(72) Inventor: ROENNOW, Troels F., Cambridge CB2 9BG (GB)
(74) Representative: Espatent Oy
(86) International application number: PCT/FI2016/050324
(87) International publication number: WO 2017/198891

(56) References cited:
- US-A1- 2013 066 644
- US-A1- 2015 205 929
- US-A1- 2016 117 471
- PETER B. NICHOL ET AL: "Co-Creation of Trust for Healthcare: The Cryptocitizen Framework for Interoperability with Blockchain", 1 January 2016 (2016-01-01), XP055578279, Retrieved from the Internet <URL:https://www.researchgate.net/publication/306013124> [retrieved on 20200623], DOI: 10.13140/RG.2.1.1545.4963
- "RoboCup 2008: RoboCup 2008: Robot Soccer World Cup XII", vol. 9977, 1 January 2016, SPRINGER INTERNATIONAL PUBLISHING, Cham, ISBN: 978-3-319-10403-4, article PAUL TAK SHING LIU: "Medical Record System Using Blockchain, Big Data and Tokenization", pages: 254 - 261, XP055573040, 032682, DOI: 10.1007/978-3-319-50011-9_20
- ANONYMOUS: "Merkle tree", WIKIPEDIA, 10 May 2016 (2016-05-10), XP055294715, Retrieved from the Internet <URL:https://en.wikipedia.org/w/index.php?title=Merkle_tree&oldid=719610335> [retrieved on 20160810]
- ANONYMOUS: "Blockchain (database)", WIKIPEDIA, 14 May 2016 (2016-05-14), XP055294728, Retrieved from the Internet <URL:https://en.wikipedia.org/w/index.php?title=Blockchain_(database)&oldid=720251964> [retrieved on 20160810]
- ANONYMOUS: "Public-key cryptography - Wikipedia, the free encyclopedia", 5 July 2015 (2015-07-05), XP055290124, Retrieved from the Internet <URL:https://en.wikipedia.org/w/index.php?title=Public-key_cryptography&oldid=670016308> [retrieved on 20160720]

## Description

### TECHNICAL FIELD

The present application generally relates to user health data verification, block-chains, distributed ledgers and cryptographic protocols. The disclosed methods, systems and devices may be used for example in data authenticity for example in health data and/or in other user privacy related application but the solution is not exclusively related to these application environments and the disclosed methods can be used elsewhere.

### BACKGROUND

This section illustrates useful background information without admission of any technique described herein representative of the state of the art.

Health data set certain requirements to measurement equipment and transferring the data. Health data is sensitive and should be treated in a way that guarantees privacy. A typical way to treat health data is to upload the data to a server. Users would feel more comfortable if their health data were stored at home. Such approach, however, creates a problem of data authenticity and a question of how a third-party can verify that the data has not been tampered with. Verification of health data could for instance be a crucial ingredient in establishing a protocol for prescription of medicine for remote consultancy with doctors, wherein the doctor should rely on the data that he or she has not taken him/herself. Likewise, for insurance purposes the verification of health data can be crucial.

In known solutions, data verification is not done and typically the devices taking health data are just trusted since there at the moment is little or no gain from faking the data. However, due to the amount of health data is increasing, for example doctors and insurance companies want to obtain data from users' personal devices and in these cases there could be a gain and temptation for somebody to fake the data.

US 2013/066644 A1 represents the closest prior art. Following publications represent state of the art: US 2015/205929 A1; US 2016/117471 A1; "Merkle tree", Wikipedia, 10 May 2016 (2016-05-10); "Blockchain (database)", Wikipedia, 14 May 2016; "Public-key cryptography - Wikipedia, the free encyclopedia", 5 July 2015 (2015-07-05); Peter B. Nichol ET AL: "Co-Creation of Trust for Healthcare: The Cryptocitizen Framework for Interoperability with Blockchain", 1 January 2016; Paul Tak Shing Liu: "Medical Record System Using Blockchain, Big Data and Tokenization", In: "RoboCup 2008: RoboCup 2008: Robot Soccer World Cup XII", 1 January 2016 (2016-01-01), Springer International Publishing, Cham 032682, vol. 9977, pages 254-261.

Thus, a technical solution is needed to solve the problem of health data authenticity and/or verification.

### SUMMARY

The scope of protection sought for various embodiments of the invention is set out by the independent claims. The embodiments and features, if any, described in this specification that do not fall under the scope of the independent claims are to be interpreted as examples useful for understanding various embodiments of the invention.

### BRIEF DESCRIPTION OF THE DRAWINGS

For a more complete understanding of example embodiments of the present invention, reference is now made to the following descriptions taken in connection with the accompanying drawings in which:
Fig. 1a shows a schematic drawing of a system of an example embodiment;
Fig. 1b shows another schematic drawing of a system of an example embodiment;
Fig. 1c shows still another schematic drawing of a system of an example embodiment;
Fig. 2 presents an example block diagram of a device in which various embodiments of the invention may be applied;
Fig. 3 shows a flow diagram illustrating a method according to an example embodiment of the invention;
Fig. 4 shows another flow diagram illustrating a method according to another example embodiment of the invention; and
Fig. 5 shows a block diagram of a server apparatus of an example embodiment.

### DETAILED DESCRIPTON OF THE DRAWINGS

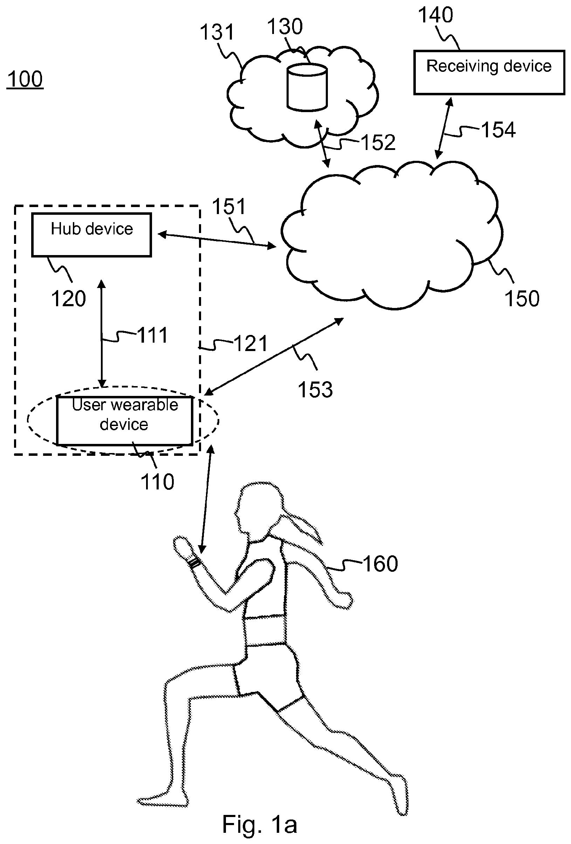

Example embodiments of the present invention and its potential advantages are understood by referring to Figs. 1a through 5 of the drawings. In this document, like reference signs denote like parts or steps.

In this document, the terms couple and connect may refer to direct contact between components or to coupling through some intervening component(s).

Fig. 1a shows a schematic drawing of a system 100 of an example embodiment.

At the minimum, the system 100 comprises a user wearable device 110 for generating user health data. The user wearable device may comprise a wrist-based device, a belt device, clothing-integrated device, skin-attached sensor, or a separate personal health device such as a thermometer or a blood pressure meter, a heart rate monitor, a blood sugar level sensor, a lactate level sensor, and an oxygen saturation sensor, for example.

In an embodiment, the user wearable device 110 generates a user health data item and hashes the user health data item using a cryptographic hashing function, to create a cryptographic hash block.

In an embodiment, the system 100 may comprise a hub device 120 for receiving and processing the user health data item. The hub device may receive the user health data item without hashing from the user wearable device 110 and carry out the user health data item hashing using a cryptographic hashing function, to create a cryptographic hash block.

In an embodiment, the hub device and the user wearable device are integrated as a single device. Alternatively, the hub device and the user wearable device are separate entities and connected via a local short-range communication interface, and the hub device and the receiving device are connected via a wide area communication interface, for example. It is also possible to arrange the hub device and the user wearable device to be releasably connectable to each other so that in one operating mode they are integrated together and in second operating mode they are separate entities.

After hashing, either the user wearable device 110 or the hub device 120 is configured to record the cryptographic hash block associated with a digital signature of the wearable device 110 to a block of a digital block-chain and transmit the user health data item to a receiving device 140 for verification.

In an embodiment, the user health data item may be encrypted before hashing. For example, asymmetrical encrypting of the user health data item may be carried out by the user wearable device 110 or the hub device 120. The encrypted user health data item may be stored to either the user wearable device 110 or the hub device 120. In case the system comprises a hub device 120, it may be beneficial to store all the user related health data items within the hub device 120 due to it is easier to arrange larger memory and storing capacity within the hub device 120 than within the user wearable device 110.

In an embodiment, the user wearable device 110 is configured to generate user health data items to the hub device 120 and the hub device takes care of encrypting the user health data items, as well as hashing the user health data items using a cryptographic hashing function, to create a cryptographic hash block, to record the cryptographic hash block associated with a digital signature of the wearable device 110 to a block of a digital block-chain and to transmit the user health data item to a receiving device 140 for verification.

In an embodiment, no hub device 120 is required in the system 100 but the user wearable device 110 is configured to generate user health data items and further takes care of encrypting the user health data items, as well as hashing the user health data items using a cryptographic hashing function, to create a cryptographic hash block, to record the cryptographic hash block associated with a digital signature of the wearable device 110 to a block of a digital block-chain and to transmit the user health data item to a receiving device 140 for verification.

In an embodiment, the hub device 120 and the user wearable device 110 are connected via a local short-range communication interface 111, and the hub device 120 and the receiving device 140 are connected via a wide area communication interface 151, 150, 154.

The local short-range communication interface 111 may comprise wired or wireless interface. The wide area communication interface may comprise a public network 150, such as Internet.

In an embodiment, the wired interface 111 comprises at least one of the following: a Universal Serial Bus (USB); and a High-Definition Multimedia Interface (HDMI). The wireless interface 111 comprises at least one of the following: a Bluetooth^{™} network; a Radio Frequency Identification (RF-ID) network; a near field communication (NFC) network; a wireless local area network; and a IEEE 802.11 network.

In an embodiment, the user wearable device 110 and the hub device 120 may be implemented as separate devices communicating with each other over a local connection 111. The local connection 111 may comprise also other wireless non-cellular connection. The wireless non-cellular connection may comprise industrial, scientific and medical (ISM) radio bands that are radio bands (portions of the radio spectrum) reserved internationally for the use of radio frequency (RF) energy for industrial, scientific and medical purposes, for example. Alternatively, the user wearable device 110 may be comprised by the hub device 120, as illustrated by an integrated apparatus 121. The apparatus 120, 121 may be for example a wrist wearable user apparatus with a sensor attached to the user 160. The integrated apparatus 121 may also correspond to a sub-system within user's home, wherein the user wearable device 110 and the hub device communicate with each other over a local connection 111.

In an embodiment, a communication interface module of at least one of the user wearable device 110 and the hub device 120 may comprise location modules for tracking location of the portable apparatus 120. Such location modules may comprise a module for providing a connection to satellite based global positioning system (e.g. GPS), a module for cellular based positioning system, a module for wireless non-cellular positioning system (e.g. Wi-Fi) or a module for hybrid positioning system, for example.

In an embodiment, the hub device 120 may be connected over a wireless or wired connection 151 to a wide area network 150, such as Internet. Router apparatuses (not shown) may be used for providing the access 151 to a wide area network 150. The access 151 may comprise cellular or non-cellular connection. The access 153 may comprise to the access 151.

In an embodiment, the system 100 comprises a server apparatus 130, which comprises a storage device for example for storing and providing user data, service data and subscriber information, over data connection 152. The service data may comprise configuration data, account creation data, user health data, and digital block-chain data, for example.

In an embodiment, a proprietary application in the user wearable device 110 or the hub device 120 may be a client application of a service whose server application is running on the server apparatus 130 of the system 100. The proprietary application may capture the user health data for the service and provide the user health data hashing, block-chain recording and transceiving for the service. In an embodiment, information from the user wearable device 110 and/or the hub device 120 to the receiving device 140 and/or the server 130 is transceived via the connections 111, 120, 150, 151, 152, 153 154 automatically. Thus the user of the devices 110, 120 may not need to do any control for the service. The system server 130 may also maintain account creation process details for the service, such as attaching new hub devices 120 or user wearable devices 110 to the system 100 as well as maintaining authorized users and devices.

In an embodiment, history data of earlier user health data, user profiles, settings and block-chains may be maintained at the server 130, for example.

The server 130 may also provide a cloud service 131 for the data of devices 110, 120, 140. Optionally, further devices may be added, such as peripheral devices for maintaining, providing or processing the hub device 120 data and communication devices for connecting the peripheral devices to the system 100.

The user wearable device 110 may operate as a biometric sensor.

The user wearable device 110 may also be applied to human skin like a temporary tattoo that can warn users exercising that they are about to become completely exhausted described, the state also described as "bonk" or "hit the wall". Thus, stamina and fitness of the user may also be monitored.

In an embodiment, the user wearable device 110, such as a lactate sensor, may comprise a bio battery offering certain advantages over conventional batteries: The bio battery recharges more quickly, uses renewable energy sources (in this case, sweat), and are safer because they do not explode or leak toxic chemicals. The sweat-powered bio battery produces energy by passing current, in the form of electrons, from an anode to a cathode. In this case, the anode contained the enzyme that removes electrons from lactate, and the cathode contained a molecule that accepts the electrons.

The hub device 120 may comprise a user interface or alternatively may not comprise user interface at all but instead the apparatus 120 is remotely operated via an external device (not shown). The hub device 120 is capable of locally executing software program code. The software program code may be a client application of a service whose server application is running on a server 130 of the system 100.

Embodiments of this invention describe how to implement a system 100 where user devices 110, 120 can store sensitive information in such a way that a verifying authority device 140 later on can confirm the authenticity of the data. The invention uses an open distributed ledger to keep a record of hashes of encrypted user data. The data may be encrypted asymmetrically such that anyone can redo the encryption of the raw data. After encryption the data is hashed and the result is added onto a ledger.

A user wearable device 110 may be located on the user, a hub device 120 may be located at user's home, and a receiving device 140 may be located at a doctor, a hospital, or an insurance company, for example. The user wearable device 110 may continuously collect and encrypt data from the user. The data may include such things a blood pressure, heart rhythm etc. The data is encrypted asymmetrically and pushed to the hub device 120 where it is stored. A hash of the asymmetrically encrypted data may then be computed and added to a block-chain. The block-chain is protected by a proof algorithm, such as proof-of-work, proof-of-stake, majority-voting or the like. The user can now at any time decrypt the data and send it to a third party 130, 140 (in practice this may be automated, and the user simply chooses which third parties may access which types of data on a continuous basis). The third party 130, 140 can then verify that this was indeed the original data that was collected by the user wearable device 110, by first asymmetrically encrypting it, computing the hash and verifying its presence on the block-chain.

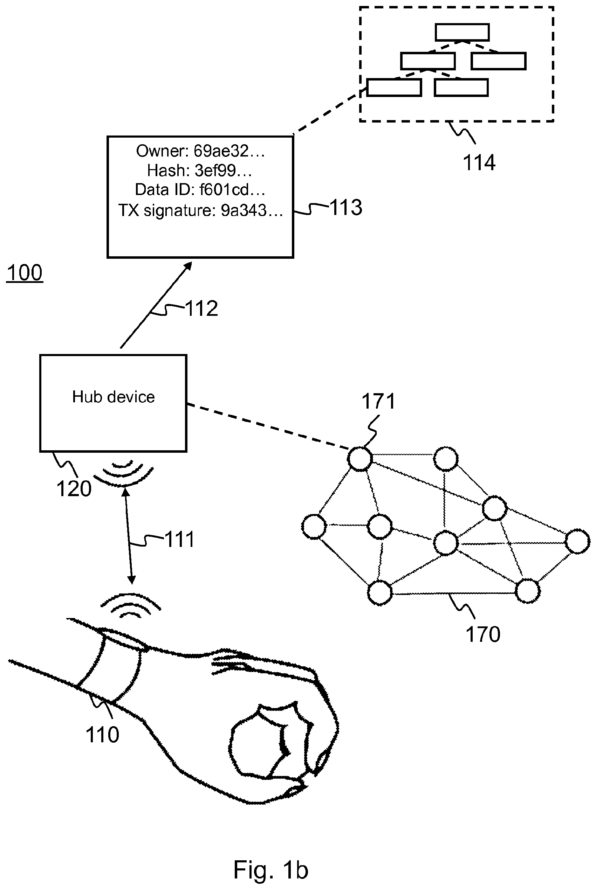

Fig. 1b shows another schematic drawing of a system 100 of an example embodiment.

In Fig. 1b it is illustrated how user health data item comprising user health related data may be generated by a user wearable device 110 and transmitted to a hub device 120 over short-range data connection 111.

The user health data item is stored at the hub device 120 that add a hash of an asymmetric encryption on to generate and transceive 112 a hash block 113. In some embodiments, the data may be stored directly on the wearable device 110. In this case the hub 120 may be optional.

The hash block 113 of the asymmetrically encrypted data may be computed and added to a digital block-chain 114. The block-chain 114 may be protected by a proof algorithm, such as proof-of-work, proof-of-stake, majority-voting or the like. The user can now at any time decrypt the data and send it to a third party within a network 170 (in practice this may be automated, and the user simply chooses which third parties may access which types of data on a continuous basis). The third party can then verify that this was indeed the original data that was collected by the user wearable device 110, by first asymmetrically encrypting it, computing the hash and verifying its presence in the block-chain. A hub device 120 (or a user wearable device 110 if the hub device is omitted) is illustrated as node 171 in the network 170 of nodes, such as a network for Internet of Things (IoT). In an embodiment, the block-chain 114 is implemented using Merkle trees. Aggregating hash values of the exchanged data in a Merkle tree is efficient, since the "root" of the Merkle tree provides a compressed digest of all individual hash values, so that the Merkle tree reduces storage requirements.

A distributed ledger is a database that can securely record user health data items for sharing across a network through entirely transparent updates of information.

The block-chain data structure 114 is an ordered, back-linked list of blocks of transactions. The block-chain 114 can be stored as a flat file, or in a simple database. Blocks 113 are linked "back," each referring to the previous block in the chain. The block-chain 114 is often visualized as a vertical stack, with blocks layered on top of each other and the first block serving as the foundation of the stack. The visualization of blocks stacked on top of each other results in the use of terms such as "height" to refer to the distance from the first block, and "top" or "tip" to refer to the most recently added block.

Although a block 113 has just one parent, it can temporarily have multiple children. Each of the children refers to the same block as its parent and contains the same (parent) hash in the "previous block hash" field. Eventually, only one child block becomes part of the block-chain 114. Even though a block 113 may have more than one child, each block can have only one parent. This is because a block has one single "previous block hash" field referencing its single parent.

Each block within the block-chain 114 is identified by a hash, generated e.g. using a SHA256 cryptographic hash algorithm on the header of the block 113. Each block 113 also references a previous block, known as the parent block, through the "previous block hash" field in the block header. In other words, each block contains the hash of its parent inside its own header. The sequence of hashes linking each block to its parent creates a chain going back all the way to the first block ever created, known as the genesis block.

In an embodiment, each block 113 in the block-chain 114 contains a summary of all the transactions in the block, using a Merkle tree. The Merkle tree 114, also known as a binary hash tree, is a data structure used for efficiently summarizing and verifying the integrity of large sets of data. Merkle trees are binary trees containing cryptographic hashes 113. The term "tree" is used in computer science to describe a branching data structure, but these trees are usually displayed upside down with the "root" at the top and the "leaves" at the bottom of a diagram, as in Fig. 1b.

In an embodiment, the Merkle tree is omitted and blocks of "transactions" are linked directly together in the block-chain 114.

The digital block-chain 114 corresponds to a distributed cryptographic ledger shared amongst all nodes participating in the network 170 of nodes, over which every successfully performed transaction is recorded.

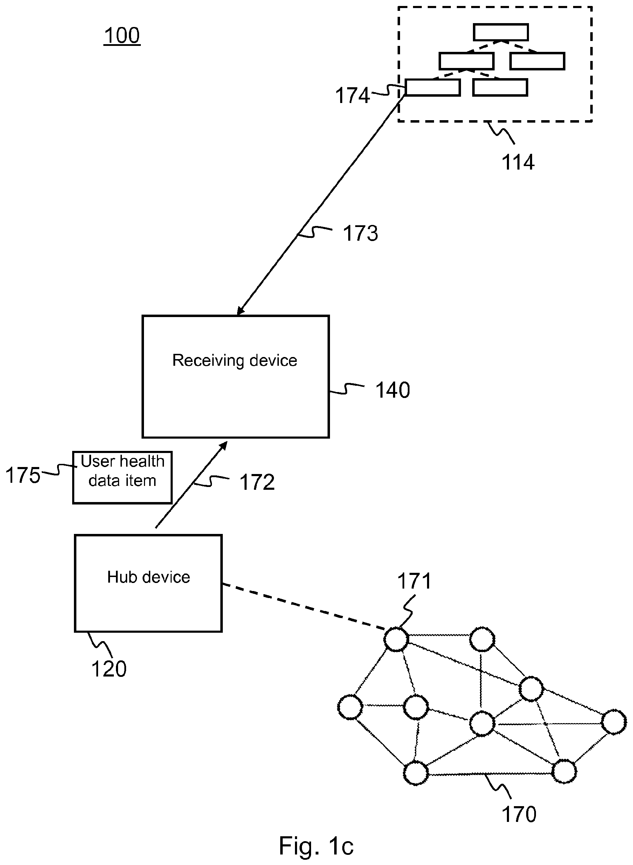

Fig. 1c shows another schematic drawing of a system 100 of an example embodiment.

In Fig. 1c it is illustrated how user health data item comprising user health related data may be verified by a receiving device 140.

The received user health data item from the hub device 120 (originating or even received from the user wearable device 110) at the receiving device 140 can be verified by the receiving device 140. The receiving device 140 may be a computer, server farm, an embedded device or special purpose circuit, for example.

In an embodiment, one may want to store the data unencrypted in which case the asymmetric encryption can be omitted in both cases. In some embodiments the user health data may be encrypted and hashed by the user wearable device 110 itself and only accepted onto the ledger 114 if a device 110 public key is verified as a certified device.

In an embodiment, a receiving device 140 receives 172 user health data item 175 either from a user wearable device 110 or from a hub device 120. The receiving device 140 hashes the user health data item 175 using a cryptographic hashing function, to create a cryptographic hash block and fetches 173 a reference cryptographic hash block 174 from the digital block-chain 114. The receiving device 140 may then compare the cryptographic hash block 175 to the fetched block 174 from the digital block-chain 114. The user health data item 175 may be verified in response to finding a matching cryptographic hash block 174 in a digital block-chain 114 based on the comparing step.

Various embodiment of the invention disclosed in the following relate to electronic circuits used in biomedical measurements. Herein, the term biomedical measurements is generally used to refer to electronic measurements of biomedical substance or organic material. The biomedical substance may be for example body or tissue of a living organism (e.g. human being) or a cell sample. Examples of biomedical measurements comprise for example electrocardiography (ECG) measurements, electrodermal activity (EDA, aka GSR galvanic skin response) measurements, body conductivity (aka bioimpedance) measurements, and impedance plethysmography (IPG) measurements, e.g. impedance cardiography (ICG).

In an embodiment, public-key cryptography, or asymmetric cryptography may be used that is any cryptographic system that uses pairs of keys: public keys that may be disseminated widely paired with private keys, which are known only to the owner. There are two functions that can be achieved: using a public key to authenticate that a message originated with a holder of the paired private key; or encrypting a message with a public key to ensure that only the holder of the paired private key can decrypt it.

In a public-key encryption system, any person/device can encrypt a message using the public key of the receiver/device, but such a message can be decrypted only with the receiver's private key. For this to work it must be computationally easy for a user to generate a public and private key-pair to be used for encryption and decryption. The strength of a public-key cryptography system relies on the degree of difficulty (computational impracticality) for a properly generated private key to be determined from its corresponding public key. Security then depends only on keeping the private key private, and the public key may be published without compromising security.

Message authentication involves hashing the message to produce a "digest," and encrypting the digest with the private key to produce a digital signature. Thereafter anyone can verify this signature by computing the hash of the message, decrypting the signature with the signer's public key, and comparing the computed digest with the decrypted digest. Equality between the digests confirms the message is unmodified since it was signed, and that the signer, and no one else, intentionally performed the signature operation - presuming the signer's private key has remained secret. The security of such procedure depends on a hash algorithm of such quality that it is computationally impossible to alter or find a substitute message that produces the same digest. The current hashing standard for encryption is SHA-2. The message itself can also be used in place of the digest.

In an embodiment, each data item is associated with its current owner's public key. When you send some data items to someone, you create a message (transaction), attaching the new owner's public key to the data item, and sign it with your private key. When this transaction is broadcast to the network, this lets everyone know that the new owner of these data items is the owner of the new key. The signature on the message verifies for everyone that the message is authentic. The complete history of transactions is kept by allowed nodes, so any allowed node can verify who is the current owner of any particular group of data items.

This complete record of transactions is kept in the block chain, which is a sequence of records called blocks. All allowed computers in the network have a copy of the block chain, which they keep updated by passing along new blocks to each other. Allowance of devices may be based on user settings. Each block contains a group of transactions that have been sent since the previous block. In order to preserve the integrity of the block chain, each block in the chain confirms the integrity of the previous one, all the way back to the first one, the genesis block. Record insertion is costly because each block must meet certain requirements that make it difficult to generate a valid block. This way, no party can overwrite previous records by just forking the chain.

To make generating health data items difficult hashing may be used. Hashing may use proof-of-work function and may use symmetric key cryptography, namely a one-way hashing function - typically either SHA1 or SHA-256).

In block-chaining, SHA-256 may be used as the underlying cryptographic hash function.

In an embodiment, a cryptographic hash function essentially takes input data, which can be of practically any size, and transforms it, in an effectively-impossible to reverse or to predict way, into a relatively compact string (in the case of SHA-256 the hash is 32 bytes). Making the slightest change to the input data changes its hash unpredictably, so nobody can create a different block of data that gives exactly the same hash. Therefore, by being given a compact hash, you can confirm that it matches only a particular input datum, and in data item the input data being a block-chain is significantly larger than the SHA-256 hash. This way, data item blocks don't have to contain serial numbers, as blocks can be identified by their hash, which serves the dual purpose of identification as well as integrity verification. An identification string that also provides its own integrity is called a self-certifying identifier.

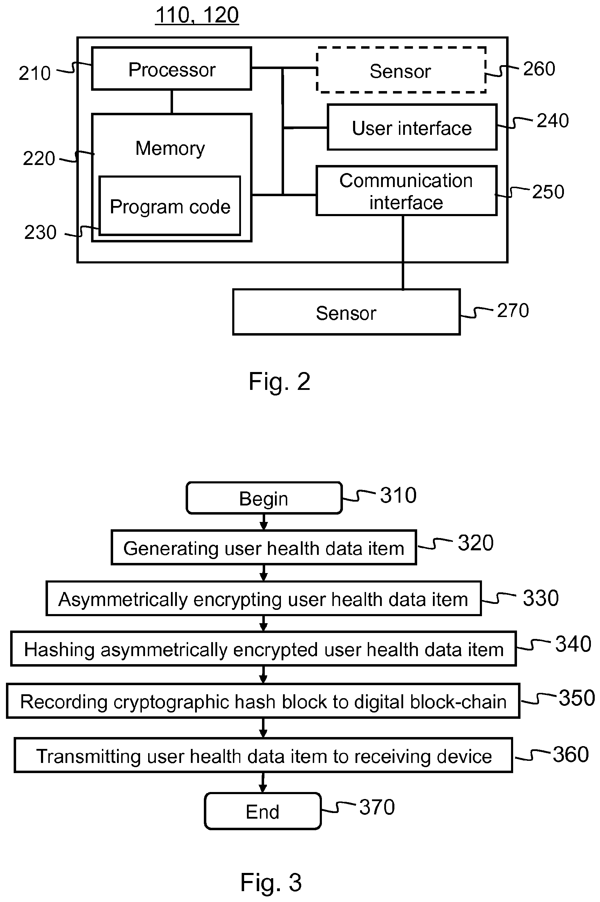

Fig. 2 presents an example block diagram of a device 110, 120 in which various embodiments of the invention may be applied. The device 120 may be a user wearable device 110 or a hub device 120 of Fig. 1a. All elements described in Fig. 2 are not necessary to be implemented in the same device 110, 120.

In an embodiment, a sensor 270 may be implemented as a separate device (e.g. user wearable device 110) communicating via the communication interface 250 with the device 120, or as an integrated sensor 260 within the device 120. The user interface 240 may be implemented also in another device connected via a communication interface 250 to the device 110, 120. Such device may comprise a mobile phone, a smart phone, or a tablet, for example. In an embodiment, the device 110, 120 may communicate with a plurality of sensors 260, 270, both internal and external sensors, and of a plurality of users.

The general structure of the device 110, 120 comprises a user interface 240, a communication interface 250, a processor 210, and a memory 220 coupled to the processor 210. The device 110, 120 further comprises software 230 stored in the memory 220 and operable to be loaded into and executed in the processor 210. The software 230 may comprise one or more software modules and can be in the form of a computer program product. Not all elements of Fig. 2 are necessary but optional for the device 110, 120, such as the user interface 240.

The processor 210 may be, e.g., a central processing unit (CPU), a microprocessor, a digital signal processor (DSP), a graphics processing unit, or the like. Fig. 2 shows one processor 210, but the device 110, 120 may comprise a plurality of processors.

The memory 220 may be for example a non-volatile or a volatile memory, such as a read-only memory (ROM), a programmable read-only memory (PROM), erasable programmable read-only memory (EPROM), a random-access memory (RAM), a flash memory, a data disk, an optical storage, a magnetic storage, a smart card, or the like. The device 110, 120 may comprise a plurality of memories. The memory 220 may be constructed as a part of the device 110, 120 or it may be inserted into a slot, port, or the like of the device 110, 120 by a user. The memory 220 may serve the sole purpose of storing data, or it may be constructed as a part of an apparatus serving other purposes, such as processing data.

The user interface 240 may comprise circuitry for receiving input from a user of the device 110, 120, e.g., via a keyboard, a touchpad, a motion sensor, a touch-screen of the device 110, 120, speech recognition circuitry, gesture recognition circuitry or an accessory device, such as a headset or a remote controller, for example. Furthermore, the user interface 240 may comprise circuitry for providing output for the user via a display, a speaker, a touch-sensitive display or a tactile feedback device, for example.

The communication interface module 250 implements at least part of data transmission. The communication interface module 250 may comprise, e.g., a wireless or a wired interface module. The wireless interface may comprise such as a WLAN, Bluetooth, infrared (IR), radio frequency identification (RF ID), NFC, GSM/GPRS, CDMA, WCDMA, or LTE (Long Term Evolution) radio module. The wired interface may comprise such as universal serial bus (USB), HDMI, SCART or RCA, for example. The communication interface module 250 may be integrated into the device 110, 120, or into an adapter, card or the like that may be inserted into a suitable slot or port of the device 110, 120. The communication interface module 250 may support one radio interface technology or a plurality of technologies. The communication interface module 250 may support one wired interface technology or a plurality of technologies. The device 110, 120 may comprise a plurality of communication interface modules 250.

In an embodiment, the communication interface module 250 may comprise location modules for tracking location of the device 110, 120. Such location modules may comprise a module for satellite based global positioning system (e.g. GPS), a module for cellular based positioning system, a module for wireless non-cellular positioning system (e.g. Wi-Fi) or a module for hybrid positioning system, for example.

A skilled person appreciates that in addition to the elements shown in Fig. 2, the device 110, 120 may comprise other elements, such as microphones, speakers, sensors, cameras, as well as additional circuitry such as input/output (I/O) circuitry, memory chips, application-specific integrated circuits (ASIC), processing circuitry for specific purposes such as source coding/decoding circuitry, channel coding/decoding circuitry, ciphering/deciphering circuitry, and the like. Additionally, the device 110, 120 may comprise a disposable or rechargeable battery (not shown) for powering when external power if external power supply is not available.

In an embodiment, the device 110, 120 comprises an additional sensor 260, 270 for providing metadata associated to the biometric information. The metadata may comprise at least one of the following: temperature information; pressure information; fingerprint information; retinal scan information; movement information; location information; and humidity information.

In an embodiment, the device 110, 120 transmits a user health data item to a receiving device for verification only in response to a pre-defined trigger. Thus, a user health data item is generated by a user wearable device, the user health data item is hashed using a cryptographic hashing function, to create a cryptographic hash block, and the cryptographic hash block associated with a digital signature of the wearable device is recorded to a block of a digital block-chain, but transmission of the user health data item to a receiving device for verification takes place in response to a trigger. Such trigger may comprise, for example, if given biometric information is present, such as fingerprint information or retina scan information detected by a sensor 260.

In an embodiment, the device 110, 120 only generates user health data item until receiving a pre-defined trigger. Thus, a user health data item (or a plurality of health data items) is/are generated by a user wearable device and in response to a trigger the user health data item is hashed using a cryptographic hashing function, to create a cryptographic hash block, and the cryptographic hash block associated with a digital signature of the wearable device is recorded to a block of a digital block-chain, and the user health data item is transmitted to a receiving device for verification. The trigger may comprise, for example, if given biometric information is present, such as fingerprint information or retina scan information detected by a sensor 260.

For instance, one can imagine a watch recording heart rate data but the data is first encrypted, hashed and signed once the watch has detected and identified a fingerprint. From this point on the watch will continuously transmit data until detached from the wrist.

In an embodiment, the device 110, 120 comprises speech or gesture recognition means. Using these means, a pre-defined phrase or a gesture may be recognized from the speech or the gesture and translated into control information for the device 110, 120.

In an embodiment, the receiving device 140 may correspond to the block structure of Fig. 2 without sensors 260, 270, for example.

Fig. 3 shows a flow diagram illustrating a method according to an example embodiment of the invention. The method begins at step 310. In step 320, a user health data item is generated by a user wearable device. In step 330, the user health data item is asymmetrically encrypted. In step 340, the encrypted user health data item is hashed using a cryptographic hashing function, to create a cryptographic hash block. In step 350, the cryptographic hash block associated with a digital signature of the wearable device is recorded to a block of a digital block-chain. In step 360, the user health data item is transmitted to a receiving device for verification. The method ends at step 370.

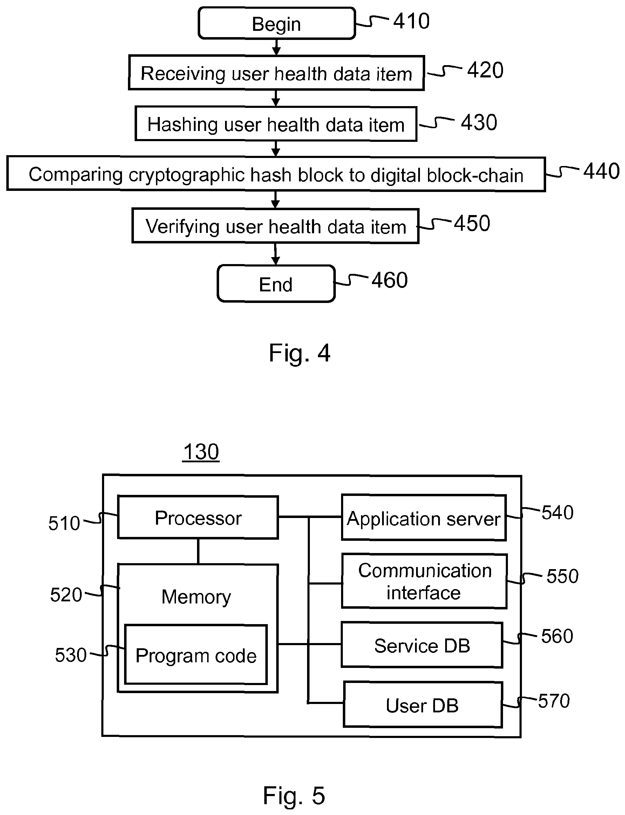

Fig. 4 shows a flow diagram illustrating a method according to an example embodiment of the invention. The method begins at step 410. In step 420, a user health data item originating from a user wearable device is received. In step 430, the user health data item is hashed using a cryptographic hashing function, to create a cryptographic hash block. In step 440, the cryptographic hash block is compared to a digital block-chain. In step 450, the user health data item is verified in response to finding a matching cryptographic hash block in a digital block-chain based on the comparing step. The method ends at step 460.

User wearable devices and sensors thereof provided in various embodiments may be used for example in heart rate detection, blood pressure detection, lactate level detection, respiration, impedance cardiography (ICG), bioelectrical impedance analysis (BIA), fingerprint detection, retinal scan detection, electrical impedance tomography (EIT) and electrodermal activity (EDA, aka GSR galvanic skin response) measurements, for example.

Fig. 5 shows a block diagram of a server apparatus 130 of an example embodiment.

The general structure of the server apparatus 130 comprises a processor 510, and a memory 520 coupled to the processor 510. The server apparatus 130 further comprises software 530 stored in the memory 520 and operable to be loaded into and executed in the processor 510. The software 530 may comprise one or more software modules and can be in the form of a computer program product.

The processor 510 may be, e.g., a central processing unit (CPU), a microprocessor, a digital signal processor (DSP), a graphics processing unit, or the like. Fig. 5 shows one processor 510, but the server apparatus 130 may comprise a plurality of processors.

The memory 520 may be for example a non-volatile or a volatile memory, such as a read-only memory (ROM), a programmable read-only memory (PROM), erasable programmable read-only memory (EPROM), a random-access memory (RAM), a flash memory, a data disk, an optical storage, a magnetic storage, a smart card, or the like. The server apparatus 130 may comprise a plurality of memories. The memory 520 may be constructed as a part of the server apparatus 130 or it may be inserted into a slot, port, or the like of the server apparatus 130 by a user. The memory 520 may serve the sole purpose of storing data, or it may be constructed as a part of an apparatus serving other purposes, such as processing data.

The communication interface module 550 implements at least part of data transmission. The communication interface module 550 may comprise, e.g., a wireless or a wired interface module. The wireless interface may comprise such as a WLAN, Bluetooth, infrared (IR), radio frequency identification (RF ID), GSM/GPRS, CDMA, WCDMA, or LTE (Long Term Evolution) radio module. The wired interface may comprise such as Ethernet or universal serial bus (USB), for example. The communication interface module 550 may be integrated into the server apparatus 130, or into an adapter, card or the like that may be inserted into a suitable slot or port of the server apparatus 130. The communication interface module 550 may support one radio interface technology or a plurality of technologies. Configuration information between the hub device 120 (or the user wearable device 110) and the system server 130 may be transceived using the communication interface 550. Similarly, account creation information between the system server 130 and a service provider may be transceived using the communication interface 550.

An application server 540 provides application services e.g. relating to the user accounts stored in a user database 570 and to the service information stored in a service database 560. The service information may comprise content information, content management information or metrics information, for example. The service information may also comprise information relating to user health data items, history data of earlier user health data items, or block-chain, for example.

A skilled person appreciates that in addition to the elements shown in Fig. 5, the server apparatus 130 may comprise other elements, such as microphones, displays, as well as additional circuitry such as input/output (I/O) circuitry, memory chips, application-specific integrated circuits (ASIC), processing circuitry for specific purposes such as source coding/decoding circuitry, channel coding/decoding circuitry, ciphering/deciphering circuitry, and the like.

Without in any way limiting the scope, interpretation, or application of the claims appearing below, a technical effect of one or more of the example embodiments disclosed herein is that an improved construction and storage of a health data block-chain representing healthcare transactions of a patient or other healthcare stakeholder. Construction and storage of the healthcare block-chain enables computing devices to quickly and efficiently verify or access user health related data, thereby improving the performance of the computing devices.

Another technical effect of one or more of the example embodiments disclosed herein is that security of sensitive user health data transmission between different devices and stakeholders is improved. Another technical effect of one or more of the example embodiments disclosed herein is that reliability of user health data is improved.

Another technical effect of one or more of the example embodiments disclosed herein is that users are allowed to store their data at home while still being able to verify the validity of the data.

Yet another technical effect of one or more of the example embodiments disclosed herein is that less complex systems are required.

Without in any way limiting the scope, interpretation, or application of the claims appearing below, a technical effect of one or more of the example embodiments disclosed herein is that an improved user health data service system is provided.

If desired, the different functions discussed herein may be performed in a different order and/or concurrently with each other. Furthermore, if desired, one or more of the before-described functions may be optional or may be combined.

It is also noted herein that while the foregoing describes example embodiments of the invention, these descriptions should not be viewed in a limiting sense. Rather, there are several variations and modifications, which may be made without departing from the scope of the present invention as defined in the appended claims.

## Claims

1. A system comprising a wearable device (110) and a hub device, (120)
the wearable device (110) comprising means for:
generating a health data item;
asymmetrically encrypting the user health data item; and
transmitting the encrypted user health data item to the hub device (120):
the wearable device (110) or the hub device (120) comprising means for hashing the encrypted user health data item using a cryptographic hashing function, to create a cryptographic hash block;
the hub device (120) further comprising means for receiving the encrypted user health data item from a user wearable device (110);
recording the cryptographic hash block associated with a digital signature of the wearable device (110) to a block of a digital block-chain; and
transmitting the encrypted user health data item to a receiving device for verification with the digital block-chain.

2. The system of claim 1, wherein the user health data item comprises information of at least one of the following:
blood pressure information;
heart rate information;
blood sugar level information;
lactate level information; and
oxygen saturation information.

3. The system of claim 1 or 2, wherein the hub device and the user wearable device are connected via a local short-range communication interface, and the hub device and the receiving device are connected via a wide area communication interface.

4. The system of claim 3, wherein the local short-range communication interface comprises wired or wireless interface.

5. The system of claim 3 or 4, wherein the wide area communication interface comprises a public network.

6. The system of any preceding claim, wherein the digital block-chain is configured to be protected by a proof algorithm comprising at least one of a proof-of-work, proof-of-stake and majority-voting algorithm.

7. The system of any preceding claim, wherein the hub device is configured to transmit the user health data item to the receiving device for verification automatically without user interaction based on settings.

8. The system of claim 7, wherein the settings comprise at least one of the following information: frequency of transmissions; continuous/timed transmission; authorized receiving devices for receiving transmissions; and types of user health data allowable for transmissions.

9. The system of any preceding claim, wherein:
the system comprises the receiving device;
the receiving device comprises means for:
receiving the encrypted user health data item originating from the user wearable device;
hashing the encrypted user health data item using the cryptographic hashing function, to create the cryptographic hash block;
comparing the created cryptographic hash block to the digital block-chain; and
verifying the user health data item in response to finding the cryptographic hash block created by the receiving device matching to the one recorded in the digital block-chain based on the comparing step.

10. A method for enabling verifying of user health data comprising:
performing by a wearable device: (110)
generating a health data item;
asymmetrically encrypting the user health data item; and
transmitting the encrypted health data item to a hub device (120);
the method further comprising
creating, by the wearable device (110) or the hub device (120), a cryptographic hash block by hashing the encrypted user health data item using a cryptographic hashing function;
and performing by the hub device (120):
receiving the user health data item from the user wearable device (110); recording the cryptographic hash block associated with a digital signature of the wearable device (110) to a block of a digital block-chain; and
transmitting the user health data item to a receiving device for verification with the digital block-chain.

## Patentansprüche

1. System, das eine tragbare Vorrichtung (110) und eine Hubvorrichtung (120) umfasst, wobei die tragbare Vorrichtung (110) Mittel für Folgendes umfasst:
Erzeugen eines Gesundheitsdatenelements;
asymmetrisches Verschlüsseln des Benutzergesundheitsdatenelements; und
Übertragen des verschlüsselten Benutzergesundheitsdatenelements zur Hubvorrichtung (120):
wobei die tragbare Vorrichtung (110) oder die Hubvorrichtung (120) Mittel zum Hashing des verschlüsselten Benutzergesundheitsdatenelements unter Verwendung einer kryptographischen Hashingfunktion umfasst, um einen kryptographischen Hashblock zu erstellen;
wobei die Hubvorrichtung (120) ferner Mittel für Folgendes umfasst
Empfangen des verschlüsselten Benutzergesundheitsdatenelements von einer von einem Benutzer tragbaren Vorrichtung (110);
Aufzeichnen des kryptographischen Hashblocks, der mit einer digitalen Signatur der tragbaren Vorrichtung (110) verknüpft ist, in einem Block einer digitalen Blockkette; und
Übertragen des verschlüsselten Benutzergesundheitsdatenelements zu einer Empfangsvorrichtung zur Verifizierung mit der digitalen Blockkette.

2. System nach Anspruch 1, wobei das Benutzergesundheitsdatenelement Informationen über mindestens eines von Folgendem umfasst:
Blutdruckinformationen;
Herzfrequenzinformationen;
Blutzuckerniveauinformationen;
Lactatniveauinformationen; und
Sauerstoffsättigungsinformationen.

3. System nach Anspruch 1 oder 2, wobei die Hubvorrichtung und die von einem Benutzer tragbare Vorrichtung via eine lokale Kurzbereichskommunikationsschnittstelle verbunden sind und die Hubvorrichtung und die Empfangsvorrichtung via eine Weitbereichskommunikationsschnittstelle verbunden sind.

4. System nach Anspruch 3, wobei die lokale Kurzbereichskommunikationsschnittstelle eine drahtgebundene oder eine drahtlose Schnittstelle umfasst.

5. System nach Anspruch 3 oder 4, wobei die Weitbereichskommunikationsschnittstelle ein öffentliches Netzwerk umfasst.

6. System nach einem der vorhergehenden Ansprüche, wobei die digitale Blockkette dazu ausgelegt ist, von einem Proofalgorithmus geschützt zu werden, der mindestens eines von einem Proof-of-Work-, einem Proof-of-Stake- und einem Majority-Voting-Algorithmus umfasst.

7. System nach einem vorhergehenden Ansprüche, wobei die Hubvorrichtung dazu ausgelegt ist, das Benutzergesundheitsdatenelement zur automatischen Verifizierung ohne eine Benutzerinteraktion auf Basis von Einstellungen zur Empfangsvorrichtung zu übertragen.

8. System nach Anspruch 7, wobei die Einstellungen mindestens eines der folgenden Informationen umfassen: Häufigkeit von Übertragungen; kontinuierliche/zeitgesteuerte Übertragung; autorisierte Empfangsvorrichtungen zum Empfangen von Übertragungen; und Arten von Benutzergesundheitsdaten, die übertragen werden dürfen.

9. System nach einem der vorhergehenden Ansprüche, wobei:
das System die Empfangsvorrichtung umfasst;
die Empfangsvorrichtung Mittel für Folgendes umfasst:
Empfangen des verschlüsselten Benutzergesundheitsdatenelements, das von der von einem Benutzer tragbaren Vorrichtung kommt;
Hashing des verschlüsselten Benutzergesundheitsdatenelements unter Verwendung der kryptographischen Hashingfunktion umfasst, um den kryptographischen Hashblock zu erstellen;
Vergleichen des erstellten kryptographischen Hashblocks mit der digitalen Blockkette; und
Verifizieren des Benutzergesundheitsdatenelements in Reaktion auf das Finden auf Basis des Vergleichsschritts, dass der kryptographische Hashblock, der von der Empfangsvorrichtung erstellt wird, mit dem in der digitalen Blockkette aufgezeichneten übereinstimmt.

10. Verfahren zum Ermöglichen des Verifizierens von Benutzergesundheitsdaten, das Folgendes umfasst:
Durchführen von Folgendem durch eine tragbare Vorrichtung (110):
Erzeugen eines Gesundheitsdatenelements;
asymmetrisches Verschlüsseln des Benutzergesundheitsdatenelements; und
Übertragen des verschlüsselten Gesundheitsdatenelements zu einer Hubvorrichtung (120);
wobei das Verfahren ferner Folgendes umfasst
Erstellen eines kryptographischen Hashblocks durch Hashing des verschlüsselten Benutzergesundheitsdatenelements unter Verwendung einer kryptographischen Hashingfunktion durch die tragbare Vorrichtung (110) oder die Hubvorrichtung (120);
und Durchführen von Folgendem durch die Hubvorrichtung (120) :
Empfangen des Benutzergesundheitsdatenelements von der von einem Benutzer tragbaren Vorrichtung (110);
Aufzeichnen des kryptographischen Hashblocks, der mit einer digitalen Signatur der tragbaren Vorrichtung (110) verknüpft ist, in einem Block einer digitalen Blockkette; und
Übertragen des Benutzergesundheitsdatenelements zu einer Empfangsvorrichtung zur Verifizierung mit der digitalen Blockkette.

## Revendications

1. Système comprenant un dispositif pouvant être porté (110) et un dispositif concentrateur (120), le dispositif pouvant être porté (110) comprenant des moyens pour :
générer un article de données de santé ;
crypter de manière asymétrique l'article de données de santé d'utilisateur ; et
transmettre l'article de données de santé d'utilisateur crypté au dispositif concentrateur (120) ;
le dispositif pouvant être porté (110) ou le dispositif concentrateur (120) comprenant des moyens pour hacher l'article de données de santé d'utilisateur crypté à l'aide d'une fonction de hachage cryptographique afin de créer un bloc de hachage cryptographique ;
le dispositif concentrateur (120) comprenant en outre des moyens pour recevoir d'un dispositif utilisateur pouvant être porté (110) l'article de données de santé d'utilisateur crypté ;
enregistrer le bloc de hachage cryptographique associé à une signature numérique du dispositif pouvant être porté (110) dans un bloc d'une chaîne de blocs numérique ; et
transmettre l'article de données de santé d'utilisateur crypté à un dispositif de réception pour vérification avec la chaîne de blocs numérique.

2. Système selon la revendication 1, dans lequel l'article de données de santé d'utilisateur comprend des informations parmi au moins les informations suivantes :
des informations de pression artérielle ;
des informations de fréquence cardiaque ;
des informations de taux de glycémie ;
des informations de taux de lactate ; et
des informations de saturation en oxygène.

3. Système selon la revendication 1 ou 2, dans lequel le dispositif concentrateur et le dispositif utilisateur pouvant être porté sont connectés via une interface de communication locale à courte portée, et le dispositif concentrateur et le dispositif de réception sont connectés via une interface de communication à large portée.

4. Système selon la revendication 3, dans lequel l'interface de communication locale à courte portée comprend une interface filaire ou sans fil.

5. Système selon la revendication 3 ou 4, dans lequel l'interface de communication à large portée comprend un réseau public.

6. Système selon l'une des revendications précédentes, dans lequel la chaîne de blocs numérique est configurée pour être protégée par un algorithme de preuve comprenant au moins un parmi un algorithme de preuve de travail, un algorithme de preuve d'enjeu et un algorithme de vote à la majorité.

7. Système selon l'une des revendications précédentes, dans lequel le dispositif concentrateur est configuré pour transmettre l'article de données de santé d'utilisateur au dispositif de réception pour vérification automatique sans interaction utilisateur sur la base de réglages.

8. Système selon la revendication 7, dans lequel les réglages comprennent des informations parmi au moins les informations suivantes : une fréquence de transmissions ; une transmission continue/temporisée ; des dispositifs de réception autorisés pour recevoir les transmissions ; et des types de données de santé d'utilisateur autorisés pour les transmissions.

9. Système selon l'une des revendications précédentes, dans lequel :
le système comprend le dispositif de réception ;
le dispositif de réception comprend des moyens pour :
recevoir du dispositif utilisateur pouvant être porté l'article de données de santé d'utilisateur crypté ;
hacher l'article de données de santé d'utilisateur crypté à l'aide d'une fonction de hachage cryptographique afin de créer le bloc de hachage cryptographique ;
comparer le bloc de hachage cryptographique créé à la chaîne de blocs numérique ; et
vérifier l'article de données de santé d'utilisateur lorsqu'il est établi que le bloc de hachage cryptographique créé par le dispositif de réception concorde avec celui enregistré dans la chaîne de blocs numérique sur la base de l'étape de comparaison.

10. Procédé permettant de vérifier de données de santé d'utilisateur comprenant les étapes suivantes :
effectuer par un dispositif pouvant être porté (110) :
générer un article de données de santé ;
crypter de manière asymétrique l'article de données de santé d'utilisateur ; et
transmettre l'article de données de santé crypté à un dispositif concentrateur (120) ;
le procédé comprenant en outre les étapes suivantes
créer, par le dispositif pouvant être porté (110) ou le dispositif concentrateur (120), un bloc de hachage cryptographique en hachant l'article de données de santé d'utilisateur crypté à l'aide d'une fonction de hachage cryptographique ;
et effectuer ce qui suit par le dispositif concentrateur (120) :
recevoir du dispositif utilisateur pouvant être porté (110) l'article de données de santé d'utilisateur ;
enregistrer le bloc de hachage cryptographique associé à une signature numérique du dispositif pouvant être porté (110) dans un bloc d'une chaîne de blocs numérique ; et
transmettre l'article de données de santé d'utilisateur à un dispositif de réception pour vérification avec la chaîne de blocs numérique.
